# EUROPEAN PATENT APPLICATION

(11) **EP 1 956 019 A1**
(43) Date of publication of application: **13.08.2008**
(21) Application number: 07100937.7
(22) Date of filing: 22.01.2007
(51) Int. Cl.: C07D 405/06, A61K 31/517, A61P 3/14

(54) **Benzoquinazoline derivatives**

(71) Applicant: Novartis AG, 4056 Basel (CH)
(72) Inventor: The designation of the inventor has not yet been filed
(74) Representative: Leon, Susanna Iris

(57) **Abstract**

A compound of formula (I) or a pharmaceutically acceptable salt or prodrug ester thereof: 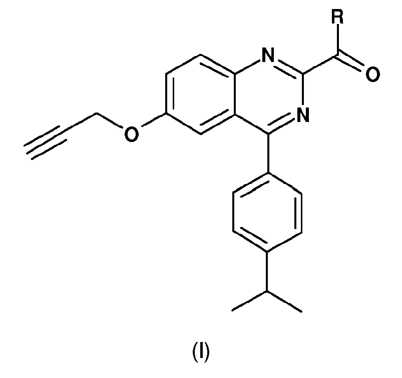 wherein R is as defined in the specification.

## Description

The present invention relates to bicyclic compounds, in particular to 2-benzoquinazoline derivatives and to pharmaceutical uses thereof.

Accordingly the invention provides a compound of formula (I) or a pharmaceutically acceptable salt thereof: 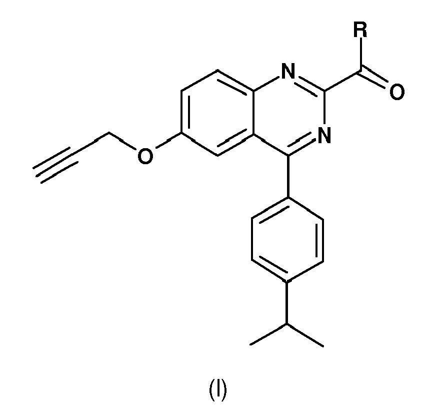 wherein -R represents a group of two fused rings -A-B
wherein A is optionally substituted heteroaryl or aryl and
B is a saturated or unsaturated 4, 5, 6 or 7 membered ring optionally containing one or more heteroatoms selected from O, N and S;
the optional substituents on R being one or more groups independently selected from oxo, cyano, halo or further optionally substituted C₁-C₆ alkyl, C₂-C₆ alkenyl, C₁-C₆ alkoxy, amino; the further optional substituents being selected from cyano, halo, C₁-C₆ alkyl, C₁-C₆ alkenyl, C₁-C₆ alkoxy, amino.

The following significances are preferred independently, collectively or in any combination or sub-combination:
(i) A is phenyl;
(ii) A is phenyl and B is fused at the 3 and 4 positions of the phenyl;
(iii) B is a 5 membered ring;
(iv) B is a 6 membered ring;
(v) B is saturated;
(vi) B is unsaturated;
(vii) B contains one heteroatom;
(viii) B contains two heteroatoms;
(ix) B contains oxygen;
(x) B contains two oxygens each of which are directly bonded to the heteroaryl or aryl ring.

For the avoidance of doubt, the terms listed below are to be understood to have the following meaning throughout the present description and claims:

The term "lower", when referring to organic radicals or compounds means a compound or radical with may be branched or unbranched with up to and including 7 carbon atoms.

A lower alkyl group may be branched, unbranched or cyclic and contains 1 to 7 carbon atoms, preferably 1 to 4 carbon atoms. Lower alkyl represents, for example: methyl, ethyl, propyl, butyl, isopropyl, isobutyl, tertiary butyl or 2,2-dimethylpropyl.

A lower alkoxy group may be branched or unbranched and contains 1 to 7 carbon atoms, preferably 1 to 6 carbon atoms. Lower alkoxy represents, for example: methoxy, ethoxy, propoxy, butoxy, isopropoxy, isobutoxy or tertiary butoxy. Lower alkoxy includes cycloalkyloxy and cycloalkyl - lower alkyloxy.

A lower alkene, alkenyl or alkenoxy group is branched or unbranched and contains 2 to 7 carbon atoms, preferably 1 to 4 carbon atoms and contains at least one carbon-carbon double bond. Lower alkene, lower alkenyl or lower alkenyloxy represents for example vinyl, prop-1-enyl, allyl, butenyl, isopropenyl or isobutenyl and the oxy equivalents thereof.

A lower akyne or alkynyl group is branched or unbranched and contains 2 to 7 carbon atoms, preferably 1 to 4 carbon atoms and contains at least one carbon-carbon triple bond. Lower alkyne or lower alkynyl or lower alkenyloxy represents for example ethynyl or propynyl.

In the present application, oxygen containing substituents, e.g. alkoxy, alkenyloxy, alkynyloxy, carbonyl, etc. encompass their sulphur containing homologues, e.g. thioalkyl, alkyl-thioalkyl, thioalkenyl, alkenyl-thioalkyl, thioalkynyl, thiocarbonyl, sulphone, sulphoxide etc.

Halo or halogen represents chloro, fluoro, bromo or iodo.

Aryl represents carbocyclic aryl or biaryl.

Carbocyclic aryl is an aromatic cyclic hydrocarbon containing from 6 to 18 ring atoms. It can be monocyclic, bicyclic or tricyclic, for example naphthyl, phenyl, or phenyl mono-, di- or trisubstituted by one, two or three substituents.

Heterocyclic aryl or heteroaryl is an aromatic monocyclic or bicyclic hydrocarbon containing from 5 to 18 ring atoms one or more of which are heteroatoms selected from O, N or S. Preferably there are one or two heteroatoms. Heterocyclic aryl represents, for example: pyridyl, indolyl, quinoxalinyl, quinolinyl, isoquinolinyl, benzothienyl, benzofuranyl, benzopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl. Heterocyclic aryl also includes such substituted radicals.

Cycloalkyl represents a cyclic hydrocarbon containing from 3 to 12 ring atoms preferably from 3 to 6 ring atoms. Cycloalkyl represents, for example: cyclopropyl, cyclobutyl, cyclopentyl, or cyclohexyl. The cycloalkyl may optionally be substituted.

Heterocycloalkyl represents a mono-, di- or tricyclic hydrocarbon which may be saturated or unsaturated and which contains one or more, preferably one to three heteroatoms selected from O, N or S. Preferably it contains between three and 18 ring atoms, more preferably between 3 and 8 ring atoms. The term heterocycloalkyl is intended also to include bridged heterocycloalkyl groups such as 3-hyroxy-8-aza-bicyclo[3.2.1]oct-8-yl.

Pharmaceutically acceptable salts include acid addition salts with conventional acids, for example mineral acids, e.g. hydrochloric acid, sulfuric or phosphoric acid, or organic acids, for example aliphatic or aromatic carboxylic or sulfonic acids, e.g. acetic, trifluoroacetic, propionic, succinic, glycolic, lactic, malic, tartaric, citric, ascorbic, maleic, fumaric, hydroxylmaleic, pyruvic, pamoic, methanesulfonic, toluenesulfonic, naphthalenesulfonic, sulfanilic or cyclohexylsulfamic acid; also amino acids, such as arginine and lysine. For compounds of the invention having acidic groups, for example a free carboxy group, pharmaceutically acceptable salts also represent metal or ammonium salts, such as alkali metal or alkaline earth metal salts, e.g. sodium, potassium, magnesium or calcium salts, as well as ammonium salts, which are formed with ammonia or suitable organic amines.

The agents of the invention which comprise free hydroxyl groups may also exist in the form of pharmaceutically acceptable, physiologically cleavable esters, and as such are included within the scope of the invention. Such pharmaceutically acceptable esters are preferably prodrug ester derivatives, such being convertible by solvolysis or cleavage under physiological conditions to the corresponding agents of the invention which comprise free hydroxyl groups. Suitable pharmaceutically acceptable prodrug esters are those derived from a carboxylic acid, a carbonic acid monoester or a carbamic acid, advantageously esters derived from an optionally substituted lower alkanoic acid or an arylcarboxylic acid.

Preferred compounds of formula (I) are:
(2,3-Dihydro-benzo[1,4]dioxin-6-yl)-[4-(4-isopropyl-phenyl)-6-propargyloxy-quinazolin-2-yl]-methanone;
Benzo[1,3]dioxol-5-yl-[4-(4-isopropyl-phenyl)-6-propargyloxy-quinazolin-2-yl]- methanone;
(2,3-Dihydro-benzofuran-5-yl)-[4-(4-isopropyl-phenyl)-6-propargyloxy-quinazolin-2-yl]-methanone.

According to a second aspect of the invention there is provided a pharmaceutical composition comprising a compound of formula (I) in association with a pharmaceutically acceptable excipient, diluent or carrier.

According to a third aspect of the invention there is provided a compound of formula (I) for promoting the release of parathyroid hormone.

It is now well established that controlled treatment of patients with parathyroid hormone (PTH) and analogues and fragments thereof can have a pronounced anabolic effect on bone formation. Thus compounds which promote PTH release, such as the compounds of the present invention may be used for preventing or treating conditions of bone which are associated with increased calcium depletion or resorption or in which stimulation of bone formation and calcium fixation in the bone is desirable.

Thus in a fourth aspect the invention includes a method for preventing or treating bone conditions which are associated with increased calcium depletion or resorption or in which stimulation of bone formation and calcium fixation in the bone is desirable in which an effective amount of a compound of formula (I) as defined above, or a pharmaceutically-acceptable and -cleavable ester, or acid addition salt thereof is administered to a patient in need of such treatment.

In an fifth aspect the invention provides a process for preparation of a compound of formula (I) in free or salt form, comprising the step of oxidizing a compound of formula IIa:

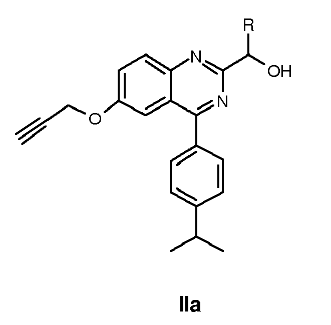

Any standard oxidation procedure may be used, for example Jones reagent under appropriate reaction conditions.

The compound of formula IIa is conveniently prepared by reacing a compound of formula II with an appropriate organometallic reagent, e.g. Grignard reagent under anhydrous conditions as illustrated:

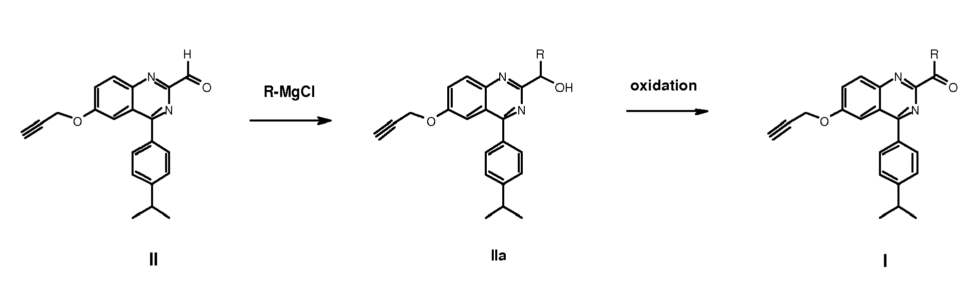

The compound of formula II may be preparted by any suitable route, for example as follows:

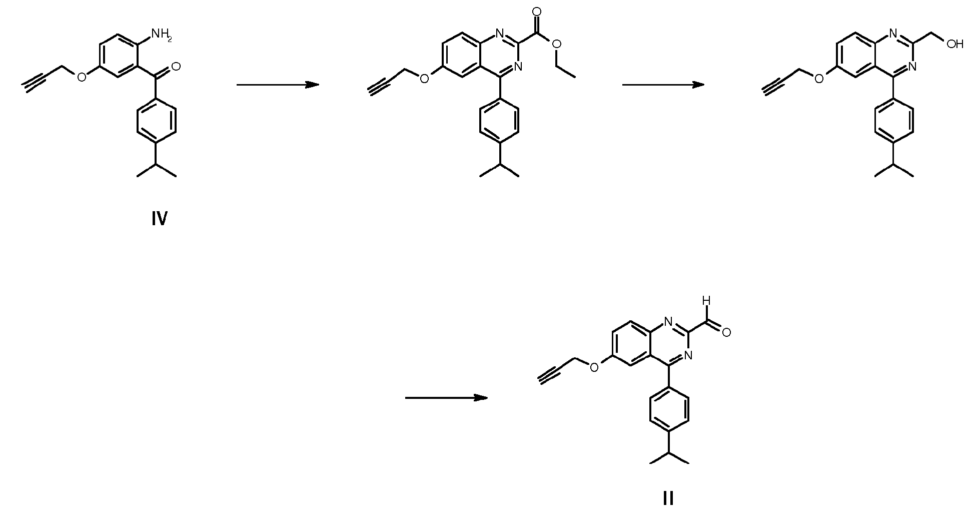

The aniline of formula IV may be prepared by any convenient route, for example as described in W02002102782 as follows: 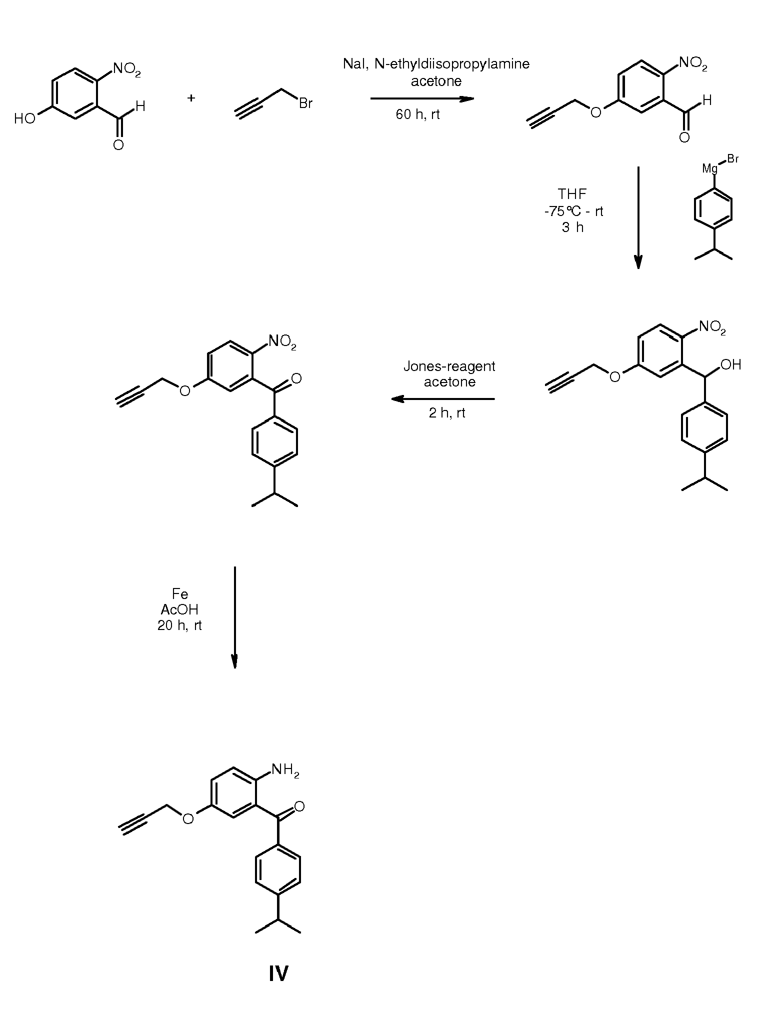 The compounds of formula (I) in free form may be converted into salt forms in conventional manner and vice-versa.

The compounds of the invention can be recovered from the reaction mixture and purified in conventional manner. Isomers, such as enantiomers, may be obtained in conventional manner, e.g. by fractional crystallization or asymmetric synthesis from corresponding asymmetrically substituted, e.g. optically active starting materials.

In a sixth aspect the invention includes the use of a compound of formula (I) in the manufacture of a medicament for preventing or treating bone conditions which are associated with increased calcium depletion or resorption or in which stimulation of bone formation and calcium fixation in the bone is desirable.

In a seventh aspect the invention provides a combination comprising a therapeutically effective amount of a compound as described above and a second drug substance selected from: calcium, a calcitonin or an analogue or derivative thereof, a steroid hormone, a partial estrogen agonist or estrogen-gestagen combination, a SERM (Selective Estrogen Receptor Modulator), vitamin D or an analogue thereof or PTH, a PTH fragment or a PTH derivative for simultaneous, separate or sequential treatment.

Agents of the invention may be prepared by processes described below, which are intended to be non-limiting examples:

The analytical HPLC conditions are as follows:
- Instrument and settings:: Agilent 110 System with G1311 A quarternary pump (0.8 ml dead volume), G1313A autosampler (1 µl injection volume), G1316A column compartment (35° C), G1315A diode array detector (detection by UV absorption at 210 nm - 250 nm wave length), G1946A mass spectrometer with APC ionization.
- Column:: Waters Symmetry C8, 50 x 2.1 mm, 3.5 µm mean particle size. flow rate 1.0 ml/min.
- Linear gradient:: 5% B in A to 95% B in A within 2.0 min.
A: water containing 5% acetonitirile and 0.1 % TFA;
B: acetonitrile containing 0.1% TFA.

### Example 1: (2,3-Dihydro-benzo[1,4]dioxin-6-yl)-[4-(4-isopropyl-phenyl)-6-propargyloxyquinazolin-2-yl]-methanone

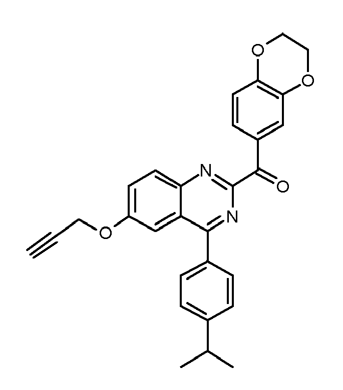

To a solution of 130 mg (0.28 mmol) of (2,3-dihydro-benzo[1,4]dioxin-6-yl)-[4-(4-isopropylphenyl)-6-propargyloxy-quinazolin-2-yl]-methanol in 5 ml of acetone are added dropwise 126 µl (0.33 mmol) 2.6 M Jones reagent. The mixture turns dark and the reaction is complete after stirring for 15 minutes at rt. After concentration i.V. the residue is distributed between ethyl acetate and water/sodium bicarbonate solution. Drying of the organic phase over anhydrous magnesium sulfate and evaporation of the solvent affords a yellow oil, which is purified by chromatography (hexanes / ethyl acetate). The product is obtained in the form of light yellow solid.
¹H-NMR (400 MHz, CDCl₃): 8.17 (d, 1H), 7.83 (d, 2H), 7.73 (d, 1H), 7.71(dd, 1H), 7.64-7.69 (m, 2H), 7.42 (d, 2H), 6.93 (d, 1H), 4.79 (d, 2H), 4.31-4.35 (m, 2H), 4.26-4.30 (m, 2H), 3.02 (hept, 1H), 2.62 (t, 1H), 1.32 (d, 6H).
MS: 465 (M+1)⁺

### Preparation of the starting material:

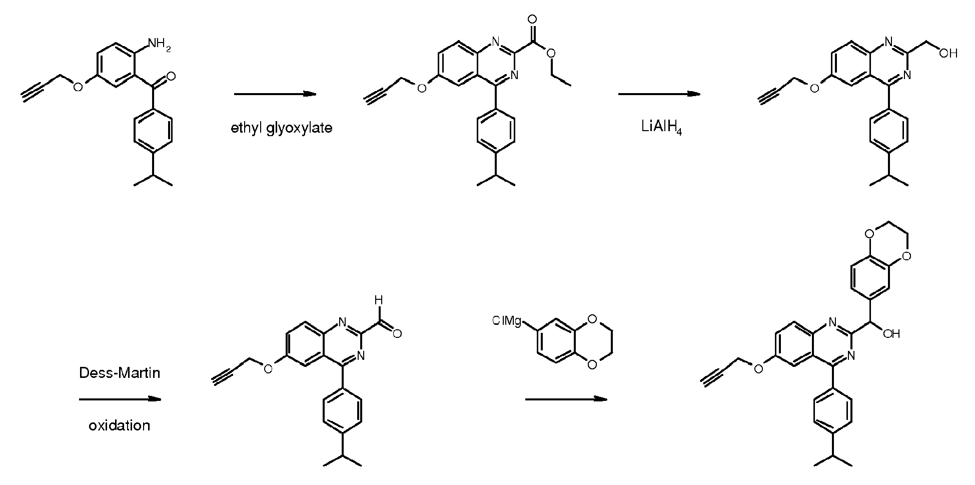

A) 4-(4-Isopropyl-phenyl)-6-propargyloxy-quinazoline-2-carboxylic acid ethyl ester To a mixture of 2 g (6.8 mmol) (2-amino-5-propargyloxy-phenyl)-(4-isopropyl-phenyl)-methanone and 1.6 g ammonium acetate are added 7 ml water and 1.4 g (6.8 mmol) ethyl glyoxylate (50% in toluene). After vigorously stirring in the presence of air for 3 days the reaction mixture is extracted with water and CH₂Cl₂. The organic layers are dried over MgSO₄ and evaporated. Purification by flash chromatography (hexane / ethyl acetate) affords 4-(4-isopropyl-phenyl)-6-propargyloxy-quinazoline-2-carboxylic acid ethyl ester.
   ¹ H-NMR (300 MHz, CDCl₃): 8.30 (d, 1 H), 7.83 (d, 2H), 7.67 (dd, 1 H), 7.65 (s, 1 H), 7.43 (d, 2H), 4.79 (d, 2H), 4.60 (q, 2H), 3.04 (hept, 1 H), 2.61 (t, 1 H), 1.50 (t, 3H), 1.34 (d, 6H) MS: 375 (M+1)⁺
B) [4-(4-Isopropyl-phenyl)-6-propargyloxy-quinazoline-2-yl-]methanol A solution of 1.0 g (2.67 mmol) of 4-(4-isopropyl-phenyl)-6-propargyloxy-quinazoline-2-carboxylic acid ethyl ester in 20 ml THF is cooled with a water/ice bath and treated with 1.6 ml 1 M lithium aluminum hydride solution. After complete addition the reaction mixture is quenched by pouring it into a saturated ammonium chloride / ethyl acetate solution.
   Extraction and concentration i.V. yields the product in the form of a yellow oil. The crude material is directly used in the following oxidation step.
C) 4-(4-Isopropyl-phenyl)-6-propargyloxy-quinazoline-2-carbaldehyde A solution of 3.9 g (11.7 mmol) of the alcohol prepared in step A in 40 ml dichloromethane is oxidized at rt with 1.1 eq Dess-Martin reagent. The mixture is filtered after stirring for 3 hrs. Distribution between ethyl acetate, water and sodium thiosulfate solution affords after concentration of the organic phases the crude aldehyde. This is purified by recrystallization from a mixture of ethyl acetate / hexanes to give a yellow-brown solid.
   ¹H-NMR (400 MHz, CDCl₃):10.29 (s, 1H), 8.25 (d, 1H), 7.82 (d, 2H), 7.67-7.72 (m, 2H), 7.45 (d, 2H), 4.80 (d, 2H), 3.04 (hept., 1H), 2.62 (t, 1H), 1.33 (d, 6H)
D) (2,3-Dihydro-benzo[1,4]dioxin-6-yl)-[4-(4-isopropyl-phenyl)-6-propargyloxy-quinazolin-2-yl]-methanol
   A suspension of 48 mg (2.0 mmol) magnesium turnings in 2 ml THF is treated with 43 mg (2.0 mmol) 6-bromo-1,4-benzodioxane solution in 2 ml THF. The reaction mixture is stirred for another 20 minutes at 60 °C, cooled to room temperature and then added to a cooled solution (5 °C) of 495 mg (1.5 mmol) of 4-(4-isopropyl-phenyl)-6-propargyloxy-quinazoline-2-carbaldehyde (in 6 ml THF). Upon complete addition stirring is continued for one hour at room temperature. The mixture is poured into 20 ml of saturated ammonium chloride solution and extracted with ethyl acetate. The crude material is purified by chromatography on silica gel (dichloromethane/ methanol) to give the alcohol in the form of a light yellow solid.
   ¹H-NMR (400 MHz, CDCl₃): 8.02 (d, 1H), 7.73 (d, 2H), 7.56-7.61 (m, 2H), 7.41 (d, 2H), 7.06-7.11 (m, 2H), 6.81 (d, 1H), 5.94 (d, 1H), 5.15 (d, 1H), 4.73 (d, 2H), 4.21 (s, 4H), 3.03 (hept, 1H), 2.58 (t, 1H), 1.33 (d, 6H).
   MS: 467 (M+1)⁺

The compounds of the following examples are prepared in an analogous manner using the appropriate starting materials:

### Example 2: Benzo[1,3]dioxol-5-yl-[4-(4-isopropyl-phenyl)-6-propargyloxy-quinazolin-2-yl]-methanone

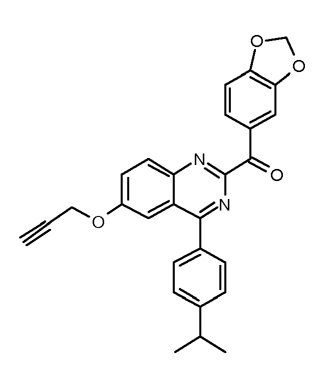

¹H-NMR (400 MHz, CDCl₃): 8.17 (d, 1H), 7.83 (d, 2H), 7.76 (dd, 1H), 7.65-7.69 (m, 3H), 7.42 (d, 2H), 6.87 (d, 1H), 6.06 (s, 2H), 4.79 (d, 2H), 3.02 (hept, 1H), 2.62 (t, 1H), 1.32 (d, 6H).
MS: 451 (M+1)⁺

### Example 3: (2,3-Dihydro-benzofuran-5-yl)-[4-(4-isopropyl-phenyl)-6-propargyloxy-quinazolin-2-yl]- methanone

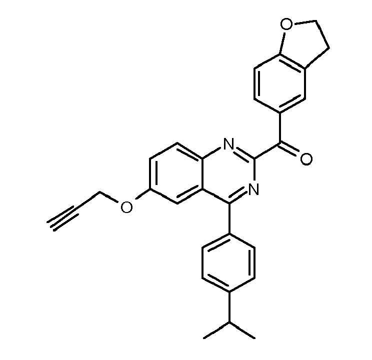

¹H-NMR (400 MHz, CDCl₃): 8.17 (d, 1H), 8.05 (s, 1H), 7.99 (d, 1H), 7.83 (d, 2H), 7.63-7.69 (m, 2H), 7.42 (d, 2H), 6.81 (d, 1H), 4.79 (d, 2H), 4.67 (t, 2H), 3.26 (t, 2H), 3.02 (hept, 1H), 2.62 (br t, 1H), 1.32 (d, 6H).
MS: 449 (M+1)⁺

The Agents of the Invention, as defined above, e.g., of formula (I), particularly as exemplified, in free or pharmaceutically acceptable acid addition salt form, exhibit pharmacological activity and are useful as pharmaceuticals, e.g. for therapy, in the treatment of diseases and conditions as hereinafter set forth.

### Assay for intracellular free calcium:

A method to determine antagonism at the PcaR consists in measuring the inhibition of intracellular calcium transients stimulated by extracellular calcium.
CCL39 fibroblasts stably transfected with human PcaR are seeded at 40'000 cells /well into 96-well Viewplates and incubated for 24 hours. Medium is then removed and replaced with fresh medium containing 2 µM Fluo-3 AM (Molecular Probes, Leiden, The Netherlands), In routine experiments, cells are incubated at 37°C, 5 % CO₂ for 1 h. Afterwards, plates are washed twice with mHBS and wells are refilled with 100 µl mHBS containing the test compounds. Incubation is continued at room temperature for 15 minutes. To record changes of intracellular free calcium, plates are transferred to fluorescence-imaging plate reader (Molecular Devices, Sunnyvale, CA, USA). A baseline consisting in 5 measurements of 0.4 seconds each (laser excitation 488 nm) is recorded. Cells are then stimulated with calcium (2.5 mM final), and fluorescence changes recorded over a period of 3 minutes.

When measured in the above assays, Agents of the Invention typically have IC₅₀s in the range from about 1000 nM down to about 1 nM or less.

It is now well established that controlled treatment of patients with parathyroid hormone (PTH) and analogues and fragments thereof can have a pronounced anabolic effect on bone formation. Thus compounds which promote PTH release, such as the Agents of the Invention may be used for preventing or treating conditions of bone which are associated with increased calcium depletion or resorption or in which stimulation of bone formation and calcium fixation in the bone is desirable.

Thus in a further aspect the invention includes a method for preventing or treating bone conditions which are associated with increased calcium depletion or resorption or in which stimulation of bone formation and calcium fixation in the bone is desirable in which an effective amount of an Agent of the Invention is administered to a patient in need of such treatment.

In a yet further aspect the invention includes a pharmaceutical composition for preventing or treating bone conditions which are associated with increased calcium depletion or resorption or in which stimulation of bone formation and calcium fixation in the bone is desirable comprising an Agent of the Invention in admixture with a pharmaceutically acceptable excipient, diluent or carrier.

Agents of the Invention are accordingly indicated for preventing or treating all bone conditions which are associated with increased calcium depletion or resorption or in which stimulation of bone formation and calcium fixation in the bone is desirable, e.g. osteoporosis of various genesis (e.g. juvenile, menopausal, post-menopausal, post-traumatic, caused by old age or by corticosteroid therapy or inactivity), fractures, osteopathy, including acute and chronic states associated with skeletal demineralisation, osteo-malacia, periodontal bone loss or bone loss due to arthritis or osteoarthritis or for treating hypoparathyroidism.

Further diseases and disorders which might be prevented or treated include e.g. seizures, stroke, head trauma, spinal cord injury, hypoxia-induced nerve cell damage such as in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, congestive heart failure; hypertension; gut motility disorders such as diarrhoea, and spastic colon and dermatological disorders, e.g. in tissue healing, for example burns, ulcerations and wounds.

The Agents of the Invention are particularly indicated for preventing or treating osteoporosis of various genesis.

For all the above uses, an indicated daily dosage is in the range from about 0.03 to about 300 mg preferably 0.03 to 30, more preferably 0.1 to 10 mg of a compound of the invention. Agents of the Invention may be administered twice a day or up to twice a week.

The Agents of the Invention may be administered in free form or in pharmaceutically acceptable salt form. Such salts may be prepared in conventional manner and exhibit the same order of activity as the free compounds. The present invention also provides a pharmaceutical composition comprising an Agent of the Invention in free base form or in pharmaceutically acceptable salt form in association with a pharmaceutically acceptable diluent or carrier. Such compositions may be formulated in conventional manner. The Agents of the Invention may be administered by any conventional route, for example parenterally e.g. in form of injectable solutions, microemulsions or suspensions, enterally, e.g. orally, for example in the form of tablets or capsules or in a transdermal, nasal or a suppository form.

According to a further embodiment of the invention, the Agents of the Invention may be employed as adjunct or adjuvant to other therapy, e.g. a therapy using a bone resorption inhibitor, for example as in osteoporosis therapy, in particular a therapy employing calcium, a calcitonin or an analogue or derivative thereof, e.g. salmon, eel or human calcitonin, a steroid hormone, e.g. an estrogen, a partial estrogen agonist or estrogen-gestagen combination, a SERM (Selective Estrogen Receptor Modulator) e.g. raloxifene, lasofoxifene, bazedoxifene, arzoxifene, FC1271, Tibolone (Livial ®), a RANKL antibody, e.g. denosumab, a cathepsin K inhibitor, vitamin D or an analogue thereof or PTH, a PTH fragment or a PTH derivative e.g. PTH (1-84), PTH (1-34), PTH (1-36), PTH (1-38), PTH (1-31)NH₂ or PTS 893.

When the Agents of the Invention are administered in conjunction with, e.g. as an adjuvant to bone resorption inhibition therapy, dosages for the co-administered inhibitor will of course vary depending on the type of inhibitor drug employed, e.g. whether it is a steroid or a calcitonin, on the condition to be treated, whether it is a curative or preventive therapy, on the regimen and so forth.

In accordance with the foregoing the present invention further provides:
a) an Agent of the Invention or a pharmaceutically acceptable salt thereof for use as a pharmaceutical;
b) a method for preventing or treating above mentioned disorders and diseases in a subject in need of such treatment, which method comprises administering to said subject an effective amount of an Agent of the Invention or a pharmaceutically acceptable salt thereof;
c) an Agent of the Invention or a pharmaceutically acceptable salt thereof for use in the preparation of a pharmaceutical composition e.g. for use in the method as in b) above.

According to a further embodiment of the invention, the Agents of the Invention may be employed as adjunct or adjuvant to other therapy, e.g. a therapy using a bone resorption inhibitor, for example as in osteoporosis therapy, in particular a therapy employing calcium, a calcitonin or an analogue or derivative thereof, e.g. salmon, eel or human calcitonin, a steroid hormone, e.g. an estrogen, a partial estrogen agonist or estrogen-gestagen combination, a SERM (Selective Estrogen Receptor Modulator) e.g. raloxifene, lasofoxifene, TSE-424, FC1271, Tibolone (Livial ®), vitamin D or an analogue thereof or PTH, a PTH fragment or a PTH derivative e.g. PTH (1-84), PTH (1-34), PTH (1-36), PTH (1-38), PTH (1-31)NH₂ or PTS 893.

When the Agents of the Invention are administered in conjunction with, e.g. as an adjuvant to bone resorption inhibition therapy, dosages for the co-administered inhibitor will of course vary depending on the type of inhibitor drug employed, e.g. whether it is a steroid or a calcitonin, on the condition to be treated, whether it is a curative or preventive therapy, on the regimen and so forth.

## Claims

1. A compound of formula (I) or a pharmaceutically acceptable salt thereof: 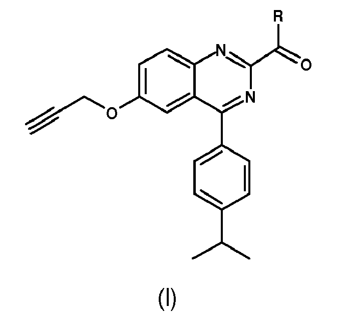 wherein -R represents a group of two fused rings -A-B
wherein A is optionally substituted heteroaryl or aryl and
B is a saturated or unsaturated 4, 5, 6 or 7 membered ring optionally containing one or more heteroatoms selected from O, N and S;
the optional substituents on R being one or more groups independently selected from oxo, cyano, halo or further optionally substituted C₁-C₆ alkyl, C₂-C₆ alkenyl, C₁-C₆ alkoxy, amino; the further optional substituents being selected from cyano, halo, C₁-C₆ alkyl, C₁-C₆ alkenyl, C₁-C₆ alkoxy, amino.

2. A compound according to claim 1 wherein A is phenyl.

3. A compound according to claim 1 or 2 wherein B is a 5 membered ring.

4. A compound according to any one of the preceding claims wherein A is phenyl and B is fused at the 3 and 4 positions of the phenyl.

5. A compound according to any one of the preceding claims wherein B contains two oxygens each of which are directly bonded to the heteroaryl or aryl ring.

6. A compound according to any one of the preceding claims selected from the following:
(2,3-Dihydro-benzo[1,4]dioxin-6-yl)-[4-(4-isopropyl-phenyl)-6-propargyloxy-quinazolin-2-yl]-methanone;
Benzo[1,3]dioxol-5-yl-[4-(4-isopropyl-phenyl)-6-propargyloxy-quinazolin-2-yl]- methanone;
(2,3-Dihydro-benzofuran-5-yl)-[4-(4-isopropyl-phenyl)-6-propargyloxy-quinazolin-2-yl]-methanone.

7. A pharmaceutical composition comprising a compound of formula (I) in association with a pharmaceutically acceptable excipient, diluent or carrier.

8. A compound of formula (I) for promoting the release of parathyroid hormone.

9. A method for preventing or treating bone conditions which are associated with increased calcium depletion or resorption or in which stimulation of bone formation and calcium fixation in the bone is desirable in which an effective amount of a compound of formula (I) as defined above, or a pharmaceutically-acceptable and -cleavable ester, or acid addition salt thereof is administered to a patient in need of such treatment.

10. A process for preparation of a compound of formula (I) in free or salt form, comprising the step of oxidizing a compound of formula IIa: 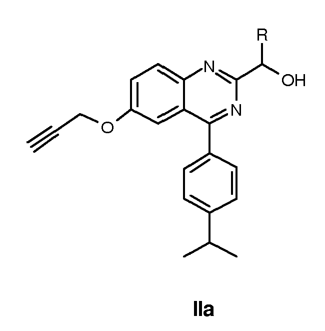 using a suitable oxidizing agent.

11. Use of a compound of formula (I) in the manufacture of a medicament for preventing or treating bone conditions which are associated with increased calcium depletion or resorption or in which stimulation of bone formation and calcium fixation in the bone is desirable.

12. A combination comprising a therapeutically effective amount of a compound according to any one of claims 1 to 6 and a second drug substance selected from: calcium, a calcitonin or an analogue or derivative thereof, a steroid hormone, a partial estrogen agonist or estrogen-gestagen combination, a SERM (Selective Estrogen Receptor Modulator), a RANKL antibody, a cathepsin K inhibitor, vitamin D or an analogue thereof or PTH, a PTH fragment or a PTH derivative for simultaneous, separate or sequential treatment.
